**Europäisches Patentamt**

**European Patent Office**

**Office européen des brevets**

(19)

(11) Veröffentlichungsnummer: **0 006 162**
**B2**

(12)

# NEUE EUROPÄISCHE PATENTSCHRIFT

(45) Veröffentlichungstag der neuen Patentschrift:
**07.01.87**

(51) Int. Cl.⁴: **C 07 C 31/18**, C 07 C 29/74

(21) Anmeldenummer: **79101686.8**

(22) Anmeldetag: **31.05.79**

(54) **Verfahren zur Gewinnung von Xylit aus Endsirupen der Xylitkristallisation.**

(30) Priorität: **14.06.78 DE 2826120**

(43) Veröffentlichungstag der Anmeldung:
**09.01.80 Patentblatt 80/1**

(45) Bekanntmachung des Hinweises auf die Patenterteilung:
**30.09.81 Patentblatt 81/39**

(45) Bekanntmachung des Hinweises auf die Entscheidung über den Einspruch:
**07.01.87 Patentblatt 87/2**

(84) Benannte Vertragsstaaten:
**AT BE CH DE FR GB IT SE**

(56) Entgegenhaltungen:
**CH - A - 541 526**
**DE - A - 2 418 800**
**DE - A - 2 418 801**
**DE - A - 2 481 800**
**DE - A - 2 710 374**
**US - A - 4 096 036**

**J. of Chromatography, Band 98, (1974), S. 70-79**

(73) Patentinhaber: **Süddeutsche Zucker-Aktiengesellschaft, Maximilianstrasse 10, D-6800 Mannheim 1 (DE)**

(72) Erfinder: **Munir, Mohammad, Dr., Wormser Strasse, D-6719 Obrigheim 5 (DE)**
Erfinder: **Schiweck, Hubert, Dr., John-F.-Kennedy-Strasse 5, D-6520 Worms (DE)**

(74) Vertreter: **Körber, Wolfhart, Dr. et al, Patentanwälte Dipl.-Ing. H. Mitscherlich Dipl.-Ing. K. Gunschmann Dr.rer.nat. W. Körber Dipl.Ing. J. Schmidt-Evers Dipl.-Ing. W. Melzer Steinsdorfstrasse 10, D-8000 München 22 (DE)**

EP 0 006 162 B2

## Beschreibung

Die Erfindung bezieht sich auf ein Verfahren zur Gewinnung von Xylit aus Endsirupen der Xylitkristallisation durch chromatographische Trennung an schwach-vernetzten stark-sauren Kationenaustauschern.

Xylit wird heute vorweigend dadurch gewonnen, indem man geeignete xylanhaltige Rohstoffe einer Säurehydrolyse unterwirft, anschliessend den entstandenen Zucker mit Wasser extrahiert, die wässrigen Hydrolysate einer Reinigung und Vollentsalzung unterzieht, sie dann hydriert und aus der hydrierten Lösung nach einer erneuten Entsalzung und Eindampfung den Xylit ein- oder mehrstufig aus Wasser auskristallisiert oder durch Fällungskristallisation mit organischen Lösungsmitteln (Methanol, Äthanol, Propanol) gewinnt.

Bei der letzten Kristallisationsstufe fällt als Endsirup eine Mutterlauge in einer Menge von 5 bis 10 Gew.% bezogen auf den Rohstoffeinsatz an, die in der Trockensubstanz immer noch etwa 30 bis 60% Xylit enthält.

Man war bisher der Auffassung, dass die Weiterkristallisation des Xylits aus dem Endsirup hauptsächlich durch deren Gehalt an Arabit, Sorbit, Galaktit, Mannit und anderen monomeren Zuckeralkoholen verhindert wird, und man entwickelte daher Verfahren, um die monomeren Zuckeralkohole voneinander trennen zu können.

Solche Verfahren sind z.B. in der DT-OS 2418800, DT-OS 2418801 und in der DT-OS 2710374 beschrieben. In den genannten Schriften erfolgt die Trennung der monomeren Zuckeralkohole an schwach-vernetzten stark-sauren Kationenaustauschern in der Calcium-, Strontium-, Aluminium- und Eisenform, wobei die Strontium-, Aluminium- und Eisenform als besonders geeignet hervorgehoben werden. In der erstgenannten Schrift sind nähere Aussagen über die dort bei der chromatographischen Trennung neben den anderen monomeren Zuckeralkoholen festgestellten unhydrierten Zucker und unbekannten Verunreinigungen nicht gemacht.

Es wurde nun überraschenderweise gefunden, dass die Kristallisation des in den Endsirupen vorliegenden Xylits nicht so sehr durch deren Gehalt an anderen monomeren Zuckeralkoholen behindert wird, sondern vielmehr durch den Gehalt derselben an Oligo- und Polysacchariden (Xylanen) und den endständig hydrierten Oligo- und Polysacchariden (Polysaccharidalkoholen), die die Viskosität der Endsirupe so weit erhöhen, dass infolge der verringerten Diffusionsgeschwindigkeit für Xylit innerhalb der Lösung und Blockierung des Xyliteinbaues in das Kristallgitter die Kristallisation zum Stillstand kommt.

Der Erfindung liegt die Aufgabe zugrunde, unter Ausnutzung der vorgenannten Erkenntnis ein Verfahren zur Gewinnung von Xylit aus Endsirupen der Xylitkristallisation vorzuschlagen. Das erfindungsgemässe Verfahren ist dadurch gekennzeichnet, dass die Trennung des Endsirups in zwei oder mehrere Fraktionen an Kationenaustauschern in der Calciumform erfolgt, von denen die erste Fraktion hauptsächlich die Polysaccharide und Polysaccharidalkohole enthält, eine weitere Fraktion vorwiegend Xylit und die nachfolgenden Fraktionen im wesentlichen die Pentite und Hexite, wobei man

a) die vorwiegend Polygosaccharide und Polysaccharidalkohole enthaltende erste Fraktion einer Säurehydrolyse unterwirft und nach Entfernen der zugesetzten Säure hydriert und aus der hydrierten Lösung den Xylit durch Kristallisation gewinnt,

b) anfallende weitere Mischfraktionen, in denen Xylit und andere Zuckeralkohole und Polysaccharide in unterschiedlichem Mengenverhältnis zueinander vorkommen, in an sich bekannter Weise einer weiteren chromatographischen Trennung unterwirft,

c) aus der vorwiegend Xylit enthaltenden nachfolgenden Fraktion den Xylit in üblicher Weise nach teilweisem oder fast vollständigem Verdampfen des Wassers durch Kühlungskristallisation oder Fällungskristallisation gewinnt.

Das erfindungsgemässe Verfahren beruht somit darauf, dass man die Di-, Tri-, Tetra- und höheren Saccharide von den monomeren Zuckeralkoholen durch Ionenaustauschchromatographie an schwach-vernetzten stark-sauren Kationenaustauschern in der Calciumform abtrennt, so dass man den Xylit aus der die monomeren Zuckeralkohole enthaltenden Fraktion direkt zur Kristallisation bringen kann.

Aus den Fraktionen, die die Di-, Tri-, Tetra- und höheren Saccharide und Saccharidalkohole enthalten, kann weiterer Xylit gewonnen werden, indem diese Fraktion auf einen Trockensubstanzgehalt von 15 bis 45% eingedampft und in Gegenwart von Säure hydrolisiert wird. Die Hydrolyse führt man z.B. in einer 0,5- bis 1%igen Schwefelsäure bei 100 bis 120°C im Druckgefäss durch. Nach 1 bis 5 Stunden ist die Hydrolyse soweit fortgeschritten, dass der grössere Teil der Saccharide in monomerer Form, vorwiegend als Xylose vorliegt. Die Schwefelsäure wird anschliessend direkt durch Perkolation der Hydrolyselösung über einen schwachbasischen Anionenaustauscher in der OH-Form entfernt oder mit Calciumcarbonat als Gips gefällt. Danach wird die neutrale Lösung bei einem Trockensubstanzgehalt von etwa 40% mit RANEY-Nickel als Katalysator unter bekannten Reaktionsbedingungen (120°C, 100 bar) hydriert und nach Abtrennen des Katalysators vollentsalzt. Die so gereinigte Lösung wird eingedampft und durch Kristallisation der Xylit gewonnen. Der Muttersirup, der nach dem Abzentrifugieren des Xylits anfällt und der wiederum ca. 40% Polysaccharide und Polysaccharidalkohole enthält, wird vor die Trennanlage zurückgeführt.

Entgegen dem bisherigen Stand des Wissens wurde überraschenderweise gefunden, dass unter den erfindungsgemässen Bedingungen der chromatographischen Trennung in Trennsäulen gefüllt mit schwach-vernetzten stark-sauren Kationenaustauschern (Divinylbenzolgehalt 3 bis 5%) die Calciumform der Austauscher besser geeignet ist als die Strontium- oder Magnesiumform.

Die erfindungsgemässen Trennbedingungen sind

Aufgabenmenge: 0,045 bis 0,055 Bettvolumen einer 50%igen Lösung des Endsirups

Elutionsmittel: Wasser mit einer Temperatur von 85 bis 95°C

Elutionstemperatur: 85 bis 95°C

Lineare Strömungsgeschwindigkeit: 2 bis 6 cm/Minute.

Zur Detektion des Eluats am Säulenausgang kann neben der Brechzahl $N_D^{27}$ für den Trockensubstanzgehalt auch die spezifische Drehung $\alpha_{546,07\,nm}^{27}$ (Polarisation) verwendet werden, da die Polysaccharide eine hohe spezifische Drehung haben, während die in Frage kommenden Pentite und Hexite optisch inaktiv sind oder nur eine geringe spezifische Drehung haben.

Die Erfindung soll beispielhaft im nachfolgenden erläutert werden, wobei ein Endsirup aufbereitet wurde, der folgende Zusammensetzung hatte:

| | |
|---|---|
| Adonit | 0,6 g/100 g Trockensubstanz |
| Arabit | 14,0 g/100 g Trockensubstanz |
| Xylit | 46,2 g/100 g Trockensubstanz |
| Mannit | 0,3 g/100 g Trockensubstanz |
| Sorbit | 2,2 g/100 g Trockensubstanz |
| Galaktit | 1,0 g/100 g Trockensubstanz |
| Oligo- und Polysaccharidalkohole | 35,7 g/100 g Trockensubstanz |

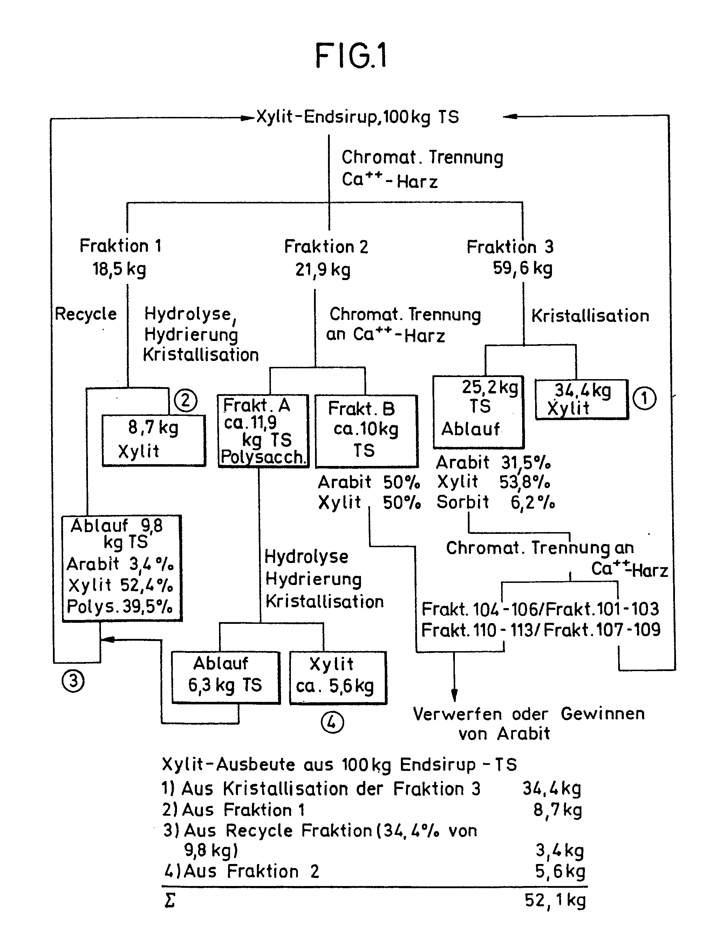

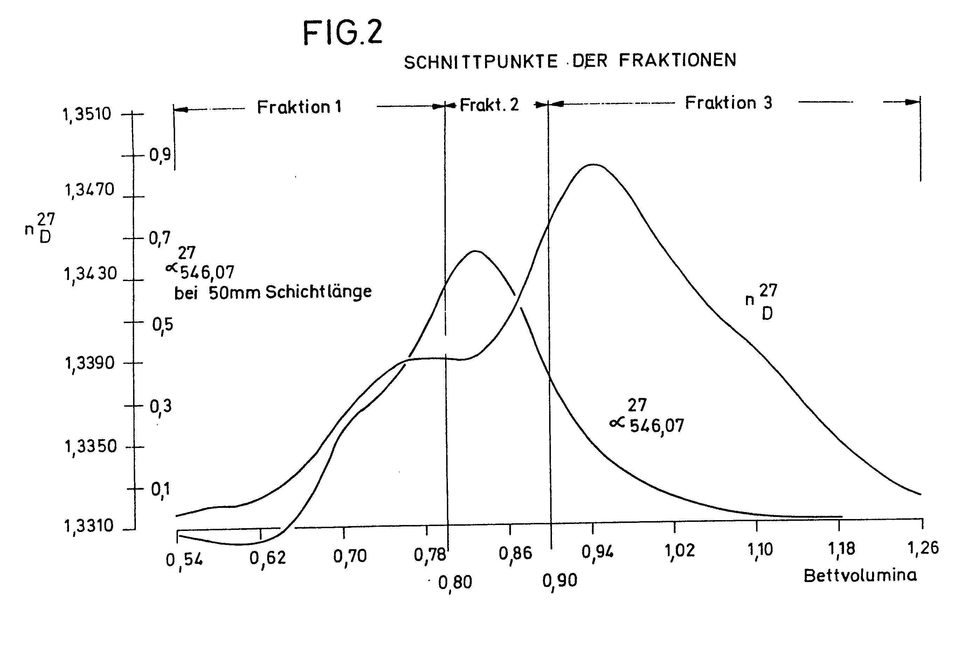

In der Abbildung 1 ist das Fliess- und Mengenschema für die Aufarbeitung von 100 kg Endsirup-Trockensubstanz obiger Zusammensetzung wiedergegeben, wobei in diesem Falle bei der chromatographischen Trennung das Gesamteluat in drei Fraktionen entsprechend Abbildung 2 unterteilt wurde.

Die Zusammensetzung der drei Fraktionen ist in nachfolgender Tabelle zusammengestellt:

| | | Fraktion 1 | Fraktion 2 | Fraktion 3 |
|---|---|---|---|---|
| Adonit | % a. TS | — | 2,1 | — |
| Arabit | % a. TS | — | 19,6 | 16,0 |
| Xylit | % a. TS | 0,3 | 2,0 | 78,4 |
| Mannit | % a. TS | — | 1,2 | — |
| Sorbit | % a. TS | — | — | 3,7 |
| Galaktit | % a. TS | — | 0,3 | 1,0 |
| Oligo- und Polysaccharidalkohole | % a. TS | 99,7 | 74,8 | 0,9 |

Zur chromatographischen Trennung wurde eine Trennanlage, bestehend aus drei hintereinandergeschalteten Säulen, 25 cm Durchmesser, 3,20 m Länge, also mit insgesamt einer Säulenlänge von 9,60 m, gefüllt mit einem stark-sauren schwachvernetzten (4% Divinylbenzol) Kationenaustauscher in der Calciumform (insgesamt ca. 500 l Harz) verwendet. Aufgegeben wurden mit einem Zyklus 15 kg Endsirup-Trockensubstanz als 50%ige Lösung, eluiert wurde mit 90° warmem Wasser mit einer Fliessgeschwindigkeit von 100 l/h.

Die Detektion des Produktstromes am Ende der Trennanlage erfolgte kontinuierlich mit einem automatischen Refraktometer und einem automatischen Polarimeter. Zu genaueren analytischen Untersuchungen wurde der Eluatstrom zusätzlich in 20-l-Fraktionen unterteilt, die nach Eindampfen auf ca. 50% Trockensubstanz auf ihre Saccharidzusammensetzung hin untersucht wurden. Unter den angegebenen Bedingungen wurden mehrere Zyklen gefahren, wie oben angegeben in drei Fraktionen unterteilt und die gleichen Fraktionen vereinigt und weiter aufgearbeitet.

Die Fraktion 1 wurde auf 25% Trockensubstanz eingedampft, soviel Schwefelsäure zugesetzt, dass eine 0,5%ige $H_2SO_4$-Lösung resultierte und das Ganze 5 Stunden bei 95°C hydrolysiert.

Das Hydrolysat wurde mit $CaCO_3$ neutralisiert, filtriert und auf 50% Trockensubstanzgehalt eingedampft. Die Lösung wurde in Gegenwart von RANEY-Nickel bei 120°C und 100 bar Wasserstoffdruck hydriert. Die Wasserstoffaufnahme war nach 2 Stunden beendet.

Nach dem Erkalten wurde RANEY-Nickel durch Zentrifugieren abgetrennt, die Lösung (die 58,1% a. TS Xylit und 2,2% a. TS Arabit enthielt) vollentsalzt und nach Eindampfung auf 85% Trockensubstanzgehalt einer Kühlungskristallisation unterworfen. Die Xylit-Ausbeute, bezogen auf die der Kristallisation unterworfene Trockensubstanzmenge, betrug 46,8%. Da 18,5% der zur Trennung gelangten ursprünglichen Xylit-Mutterlauge-Trockensubstanz in die Fraktion 1 gegangen waren, betrug die zusätzliche Xylit-Ausbeute 8,7% der ursprünglichen Xylit-Mutterlauge-Trokkensubstanz.

Die nach Abtrennen der Kristalle erhaltene Mutterlauge hatte folgende Zusammensetzung:

| | |
|---|---|
| Adonit | 1,0% a. TS |
| Arabit | 3,4% a. TS |
| Xylit | 52,4% a. TS |
| Mannit | 1,0% a. TS |
| Sorbit | 1,0% a. TS |
| Galaktit | 1,7% a. TS |
| Oligo- u. Polysaccharidalkohole | 39,5% a. TS |

Der hohe Gehalt an Oligo- und Polysacchariden erhöht die Viskosität der Lösung und verhindert die Kristallisation von Xylit. Dieser Ablauf wird zu einer weiteren Trennung zurückgeführt und dadurch eine weitere Menge Xylit gewonnen. So ist es möglich, aus Fraktion 1 ca. 70% des Trockensubstanzgehaltes als Xylit zu gewinnen.

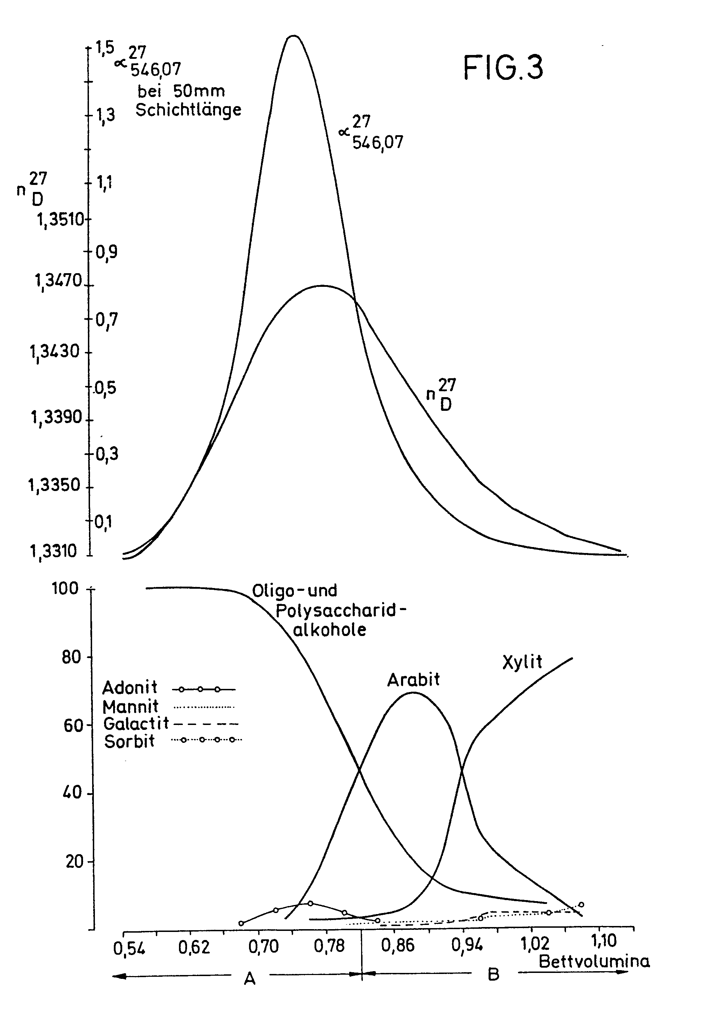

Wie aus der Zusammensetzung der Fraktion 2 hervorgeht, lässt sich direkt aus dieser kein Xylit gewinnen. Es wurden daher die Fraktionen 2 mehrerer Zyklen vereinigt, auf 50% TS eingedampft und auf der oben beschriebenen Trennanlage unter denselben Bedingungen nochmals einer chromatographischen Trennung unterworfen. Das Ergebnis dieser Trennung ist in Abbildung 3 zusammengestellt. Wie man aus der Abbildung erkennt, erhält man eine Fraktion A, die praktisch nur aus Oligo- und Polysacchariden besteht, und eine Fraktion B, in der Arabit und Xylit etwa in gleichen Teilen vorliegen, wenn man die Trennung des Eluats bei etwa 0,82 Bettvolumina durchführt.

Die Fraktion A wird analog der Fraktion 1 alleine oder mit dieser zusammen aufgearbeitet.

Die Fraktion B kann durch eine weitere chromatographische Trennung in Arabit und Xylit zerlegt werden.

Die Fraktion 3 aus mehreren Zyklen wurde gesammelt, auf 85% Trockensubstanzgehalt eingedampft und zur Kristallisation in eine Kühlmaische gegeben. Der Xylit kristallisierte sehr leicht aus und liess sich in einer Siebkorbzentrifuge leicht von der Mutterlauge abtrennen. Die Kristallausbeute, bezogen auf die der Kristallisation unterworfene Gesamttrockensubstanzmenge, betrug 57,8%. Somit konnten 34,4% der ursprünglichen Xylit-Endsirup-Trockensubstanz als kristallines Xylit gewonnen werden.

Der nach Abtrennen anfallende Endsirup hatte folgende Zusammensetzung:

| | |
|---|---|
| Adonit | – % a. TS |
| Arabit | 31,5% a. TS |
| Xylit | 53,8% a. TS |
| Mannit | – % a. TS |
| Sorbit | 6,2% a. TS |
| Galaktit | 2,0% a. TS |
| Oligo- und Polysaccharidalkohole | 6,3% a. TS |

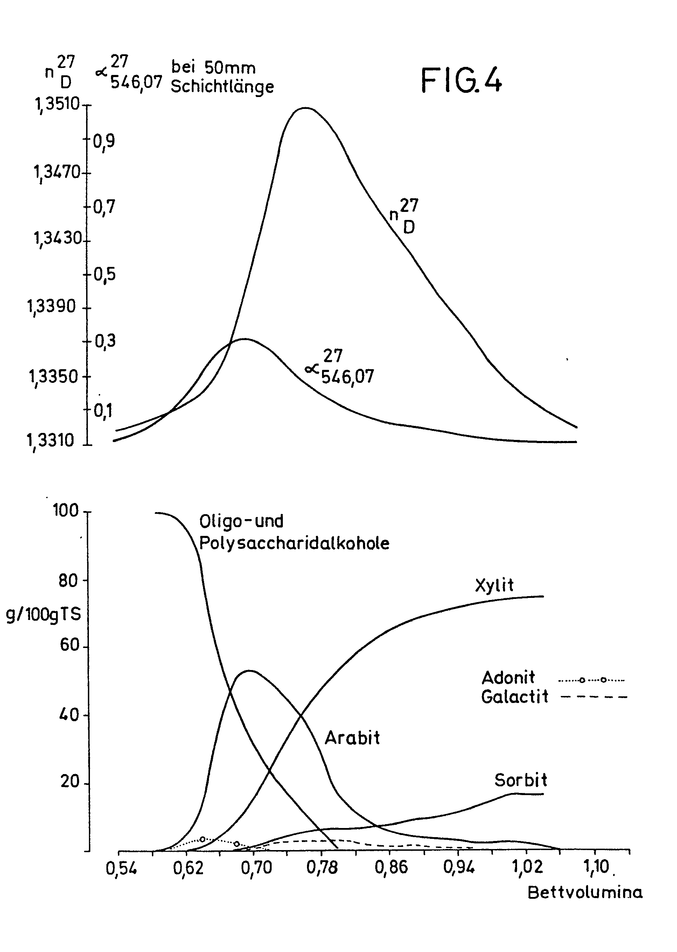

Dieser Endsirup wurde einer weiteren chromatographischen Trennung unter den oben beschriebenen Bedingungen unterworfen und in die Fraktionen 101 bis 114 zerlegt. In der Abbildung 4 ist die Fraktionsaufteilung und in der Tabelle 1, S. 12, die Saccharidzusammensetzung der einzelnen Fraktionen zusammengestellt. Wie in der Abbildung 1 dargestellt, werden die Fraktionen 104 bis 106 sowie 110 bis 113 verworfen oder dienen als Ausgangsmaterial für die Gewinnung von Arabit, während die Fraktionen 101 bis 103 und 107 bis 109 zurückgeführt werden.

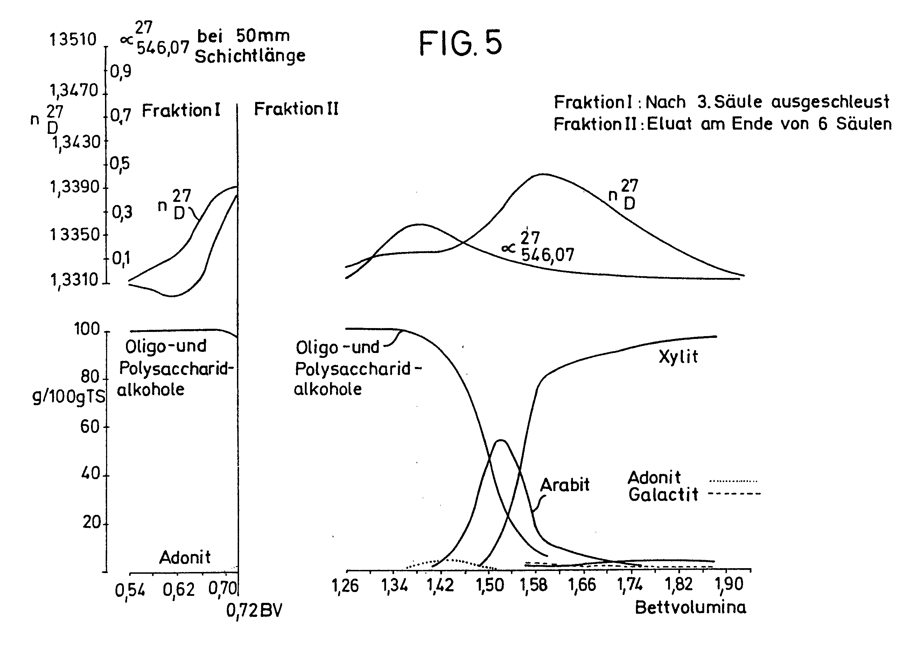

Schaltet man nicht, wie im vorhergehenden Beispiel angegeben, 3 sondern 6 Trennsäulen hintereinander und schleust einen Teil der Polysaccharide bereits am Ende der 3. Trennsäule aus dem System heraus, dann gelingt es, eine praktisch vollständige Abtrennung der Polysaccharide in einem Trenngang zu erreichen, wie aus der Abbildung 5 hervorgeht. Auch kann man bei der verlängerten Trennsäulenlänge eine gute Abtrennung des Arabits vom Xylit erreichen.

Aufgrund der gewonnenen Erkenntnisse schien es geboten, zu versuchen, die Hydrolyse der Polysaccharide bereits vor der chromatographischen Trennung durchzuführen. Versuche mit einer Säurehydrolyse in dieser Richtung führten zu dem Ergebnis, dass auf diese Weise nur eine ungenügende Hydrolyse zu erreichen ist. So wurde z.B. bei einer Säurehydrolyse in 0,5%iger Schwefelsäure bei 25% Trockensubstanzgehalt in der Hydrolyselösung bei 100°C bereits nach 2 Stunden ein Reduktionswert von 12,6%, bezogen auf Trockensubstanz und Glucose = 100%, gefunden, der sich nur geringfügig auf 16% innerhalb von 22 Stunden erhöhte, jedoch nicht den theoretischen Wert von ca. 35% erreichte. Nach Hydrierung und entsprechender Aufbereitung der Hydrolysenlösung gelang es wohl, einen Teil des Xylits zur Kristallisation zu bringen, die zusätzliche Xylit-Ausbeute betrug jedoch nur 10 bis 20%, bezogen auf die eingesetzte Trockensubstanz, und blieb somit weit unter dem Wert für das oben beschriebene Verfahren zurück.

Tabelle I

| Fraktion | 101 | 102 | 103 | 104 | 105 | 106 | 107 | 108 | 109 | 110 | 111 | 112 | 113 |
|---|---|---|---|---|---|---|---|---|---|---|---|---|---|
| Bettvolumina | 0,56 | 0,60 | 0,64 | 0,68 | 0,72 | 0,76 | 0,80 | 0,84 | 0,88 | 0,92 | 0,96 | 1,00 | 1,04 |
| Adonit | – | – | 3,2 | 1,9 | – | – | – | – | – | – | – | – | – |
| Arabit | – | 0,1 | 14,7 | 50,5 | 49,3 | 39,1 | 15,8 | 7,8 | 4,9 | 2,5 | 2,0 | 2,0 | 1,2 |
| Xylit | – | – | 0,9 | 19,2 | 28,2 | 41,8 | 71,7 | 83,9 | 84,8 | 86,4 | 84,5 | 80,9 | 81,8 |
| Mannit | – | 0,1 | 0,8 | 1,6 | – | – | – | – | – | – | – | – | – |
| Sorbit | – | – | – | – | 2,6 | 5,0 | 5,1 | 7,0 | 8,8 | 10,0 | 12,8 | 16,4 | 16,4 |
| Galactit | – | – | – | 1,4 | 2,1 | 2,5 | 3,0 | 1,2 | 1,5 | 0,6 | 0,5 | 0,7 | – |
| Σ | – | 0,2 | 19,6 | 74,6 | 82,2 | 88,4 | 95,6 | 99,9 | 100 | 99,5 | 99,8 | 100 | 99,4 |
| Oligo- und Polysaccharidalkohole | 100 | 99,8 | 80,4 | 25,4 | 17,8 | 11,3 | 4,6 | – | – | – | – | – | – |
| Σ | 100 | 100 | 100 | 100 | 100 | 99,7 | 100,2 | 99,9 | 100 | 99,5 | 99,8 | 100 | 99,4 |

## Patentansprüche

1. Verfahren zur Gewinnung von Xylit aus Endsirupen der Xylitkristallisation durch chromatographische Trennung an schwach-vernetzten stark-sauren Kationenaustauschern, dadurch gekennzeichnet, dass die Trennung des Endsirups in 2 oder mehrere Fraktionen zu Kationenaustauschern in der Calciumform mit salzfreiem Wasser als Elutionsmittel erfolgt, von denen die erste Fraktion hauptsächlich die Polysaccharide und Polysaccharidalkohole enthält, weitere Mischfraktionen, im wesentlichen die Pentite und Hexite, und die nachfolgende Fraktion, vorwiegend Xylit, wobei man

a) die vorwiegend Polysaccharide und Polysaccharidalkohole enthaltende erste Fraktion einer Säurehydrolyse unterwirft und nach Entfernen der zugesetzten Säure hydriert und aus der hydrierten Lösung den Xylit durch Kristallisation gewinnt,

b) anfallende weitere Mischfraktionen, in denen Xylit und andere Zuckeralkohole und Polysaccharide in unterschiedlichem Mengenverhältnis zueinander vorkommen, in an sich bekannter Weise einer weiteren chromatographischen Trennung unterwirft,

c) aus der vorwiegend Xylit enthaltenden nachfolgenden Fraktion den Xylit in üblicher Weise nach teilweisem oder fast vollständigem Verdampfen des Wassers durch Kühlungskristallisation oder Fällungskristallisation gewinnt.

2. Verfahren nach Anspruch 1, dadurch gekennzeichnet, dass die chromatographische Trennung bei 85 bis 90°C und einer linearen Strömungsgeschwindigkeit von 2 bis 6 cm/Minute erfolgt.

3. Verfahren nach Anspruch 1, dadurch gekennzeichnet, dass die Polysaccharide/Polysaccharidalkohole enthaltende erste Fraktion bei einem Trockensubstanzgehalt von 15 bis 40 Gew.% in einer 0,5- bis 1%igen Schwefelsäure bei 100 bis 120°C drucklos oder unter Druck in einer Zeit von 1 bis 5 Stunden hydrolysiert wird.

4. Verfahren nach einem der vorhergehenden Ansprüche, dadurch gekennzeichnet, dass die bei den einzelnen Stufen der chromatographischen Trennung der Mischfraktionen anfallenden Muttersirupe, nach Abtrennung der Xylitkristalle, wieder vor die chromatographische Trennung zurückgeführt werden.

## Claims

1. A process for recovering xylitol from end sirups of the xylitol-crystallization by means of chromatographic separation of weakly crosslinked, strongly acidic cation exchangers characterized by splitting the end sirups into two or more fractions in cation exchangers in the calcium salt form with deionized water as eluent, the first fraction containing predominantly the polysaccarides and polysaccharide-alcohols, further mixed fractions containing in the main the pentitols and hexitols, and the subsequent fraction containing in the main xylitol, by

a) subjecting the first fraction predominantly containing polysaccharides and polysaccharide-alcohols to an acid hydrolysis, hydrogenating it after the removal of the added acid, and recovering xylitol from the hydrogenated solution by crystallization,

b) subjecting in a manner known per se resulting subsequent mixed fractions, in which xylitol and other sugar alcohols and polysaccharides are present in varying proportions to further chromatographic separation,

c) recovering xylitol from a fraction predominantly containing xylitol in the usual manner after a partially or nearly complete evaporization of the water by cooling crystallization or precipitation crystallization.

2. A process according to claim 1 characterized in that the chromatographic separation is accomplished at a temperature from 85°–90°C and at a linear flow rate of 2–6 cm/minute.

3. A process according to claim 1 characterized in that the first fraction containing polysaccharides/polysaccharide-alcohols having a dry substance content of 15 to 40% by weight is hydrolized in a 0,5%–1% sulfuric acid at a temperature from 100° to 120°C in the absence or presence of pressure for a time between 1 to 5 hours.

4. A process according to one of the preceding claims characterized in that the mother sirups occurring at the individual stages of the chromatographic separation of the mixed fractions, after the separation of the xylitol crystals, are again subjected to the chromatographic separation.

## Revendications

1. Procédé pour extraire du xylitol des sirops résiduaires de la cristallisation de xylitol, par la séparation chromatographique sur des échangeurs de cations faiblement réticulés et fortement acides, caractérisé en ce que la séparation du dernier sirop-mère en deux ou plusieurs fractions se fait sur des échangeurs de cations sous forme calcique, avec de l'eau déminéralisée comme agent d'élution, la première fraction (1) contenant principalement les polysaccharides et les alcools de polysaccharides, les autres fractions mixtes (2) contenant surtout le pentol et l'hexol tandis que la fraction suivante contient pour l'essentiel du xylitol et où:

a) la première fraction (1) qui contient principalement des polysaccharides et des alcools de polysaccharides, subit une hydrolyse acide, et elle est hydrogénée après l'élimination de l'acide ajouté et le xylitol est séparé par cristallisation de la solution hydrogénée;

b) les fractions mixtes suivantes obtenues (2, 3) qui contiennent du xylitol et d'autres alcools de sucre et polysaccharides en des proportions quantitatives variables, sont soumises, de manière connue, à une nouvelle séparation chromatographique;

c) à partir de la fraction suivante, contenant principalement du xylitol, ce dernier est obtenu, de manière usuelle, après évaporation partielle ou presque totale de l'eau, par cristallisation par refroidissement ou par cristallisation par précipitation.

2. Procédé selon la revendication 1, caractérisé en ce que la séparation chromatographique est effectuée à 85°–90°C et avec une vitesse d'écoulement linéaire comprise entre 2 et 6 cm/minute.

3. Procédé selon la revendication 1, caractérisé en ce que la première fraction (1) contenant les polysaccharides et alcools de polysaccharides, concentrée à une teneur en matière sèche d'entre 15 et 40% en poids, est hydrolysée dans de l'acide sulfurique à entre 0,5 et 1%, à une température comprise entre 100 et 120°C, sans pression ou sous pression, pendant une durée de 1 à 5 heures.

4. Procédé selon l'une quelconque des revendications précédentes, caractérisé en ce que les liqueurs-mères obtenues à chaque étage de séparation chromatographique des fractions mixtes (2, 3) sont recyclées en amont de la zone de séparation chromatographique.

0 006 162

# FIG.1

Xylit-Ausbeute aus 100 kg Endsirup – TS

| | |
|---|---|
| 1) Aus Kristallisation der Fraktion 3 | 34,4 kg |
| 2) Aus Fraktion 1 | 8,7 kg |
| 3) Aus Recycle Fraktion (34,4 % von 9,8 kg) | 3,4 kg |
| 4) Aus Fraktion 2 | 5,6 kg |
| $\Sigma$ | 52,1 kg |

# FIG.2

SCHNITTPUNKTE · DER FRAKTIONEN

FIG.3

FIG.4

$n_D^{27}$   $\alpha_{546,07}^{27}$ bei 50mm Schichtlänge

FIG. 5

$\alpha_{546,07}^{27}$ bei 50mm Schichtlänge

$n_D^{27}$

Fraktion I    Fraktion II

Fraktion I : Nach 3. Säule ausgeschleust
Fraktion II : Eluat am Ende von 6 Säulen

$n_D^{27}$

$\alpha_{546,07}^{27}$

Oligo-und Polysaccharid-alkohole

Oligo-und Polysaccharid-alkohole

Xylit

Adonit

Arabit

Adonit ............
Galactit --------

g/100gTS

0,54    0,62    0,70    0,72 BV

1,26  1,34  1,42  1,50  1,58  1,66  1,74  1,82  1,90    Bettvolumina

0 006 162